# EUROPEAN PATENT SPECIFICATION

(11) **EP 1 375 488 B1**
(45) Date of publication and mention of the grant of the patent: **02.08.2006**
(21) Application number: 03016090.7
(22) Date of filing: 02.09.1999
(51) Int. Cl.: C07D 233/02, C07D 263/06, C07D 265/30, C07D 303/17, C07D 303/31, C07D 307/77, C07D 405/14, C07D 407/14, C07D 413/14, C07D 493/22, C07D 493/14, A61K 31/34, A61P 29/00, C07D 307/00, C07D 303/00

(54) **Triptolide prodrugs having high aqueous solubility**
Triptolid-Prodrugs mit hoher Wasserlöslichkeit
Promedicaments de triptolodes à solubilite dans l'eau elevée

(30) Priority: 02.09.1998 US 98809 P
(43) Date of publication of application: 02.01.2004
(62) Divisional of application: 99949582.3
(73) Proprietor: PHARMAGENESIS, INC., Palo Alto, CA 94304 (US)
(72) Inventor: Musser, John H., San Carlos, California 94070 (US)
(74) Representative: Vossius & Partner

(56) References cited:
- WO-A-97/31920
- DATABASE WPI Section Ch, Week 199142 Derwent Publications Ltd., London, GB; Class B02, AN 1991-305574 XP002181288 -& JP 03 178977 A (CHUGOKU IGAKU KAGAK), 2 August 1991 (1991-08-02)
- DE QUAN YU, ET AL.: "Chemical Transformation of Triptolide" CHINESE CHEMICAL LETTERS, vol. 2, no. 12, 1991, pages 937-940, XP001025887
- DAN Y ET AL: "Studies on Triepoxide Analogs of Triptolide" TETRAHEDRON LETTERS, ELSEVIER SCIENCE PUBLISHERS, AMSTERDAM, NL, vol. 38, no. 39, 29 September 1997 (1997-09-29), pages 6865-6868, XP004090305 ISSN: 0040-4039

## Description

The present invention relates to compounds useful as immunosuppressive and anti-inflammatory agents. The compounds have good aqueous solubility and convert to biologically active compounds *in vivo*.

### References

Ferrier, R.J. in CARBOHYDRATE CHEMISTRY, Kennedy, J.F., Ed., Clarendon Press, Oxford (1990).
Garver, L.C. *et al., J. Am. Chem. Soc.* 104:867 (1982).
Gleichmann, E. *et al., Immunol. Today* 5:324 (1984).
Hormi, O.E.O. and Nasman, J.H., *Syn. Commun.* **16**:69 (1986).
Kocienski, P.J., PROTECTING GROUPS, Georg Thieme Verlag, Stuttgart (1994).
Korngold, R. and Sprent, J., *J. Exp. Med.* 148:1687 (1978).
Kupchan, S.M. *et al., J. Am. Chem. Soc.* 94:7194 (1972).
Kupchan, S.M. *et al.,* U.S. Patent No. 3,005,108 (1977).
Lipsky, P.E. *et al*., U.S. Patent No. 5,294,443 (1994).
Ma, P-C. *et al., J. Chin. Pharm. Sci*. 1:12 (1992).
Mori, S. *et al*., *Tetrahedron* 47(27):5051-5070 (1991).
Morris, R.E., *Transplant Proc.* 23(6):2722-2724 (1991).
Morris, R.E. *et al., Transplant Proc.* 23(1):238-240 (1991).
Murase, N. *et al., Transplantation* 55:701 (1993).
Ono and Lindsey, *J*. *Thor. Cardiovasc. Surg.* 57(2):225-29 (1969).
Pu, L. *et al., Zhongguo Yaoli Xuebao* 11:76 (1990).
Wang, J. and Morris, R.E., *Transplantation Proc.* 23:699 (1991).
Yu *et al., Acta Pharmaceutica Sinica* 27(11):830-836 (1992).
Zheng, J. *et al., Zhongguo Yixue Kexueyuan Xuebao* 13:391 (1991).
Zheng, J. *et al., Zhongguo Yixue Kexueyuan Xuebao* 16:24 (1994).

Immunosuppressive agents are widely used in the treatment of autoimmune disease and in treating or preventing transplantation rejection, including the treatment of graft-versus-host disease (GVHD), a condition in which transplanted marrow cells attack the recipient's cells. Common immunosuppressive agents include azathioprine, corticosteroids, cyclophosphamide, methotrexate, 6-mercaptopurine, vincristine, and cyclosporin A. In general, none of these drugs are completely effective, and most are limited by severe toxicity. For example, cyclosporin A, a widely used agent, is significantly toxic to the kidney. In addition, doses needed for effective treatment may increase the patient's susceptibility to infection by a variety of opportunistic invaders.

A number of compounds isolated from the Chinese medicinal plant *Tripterygium wilfordii* (TW) have been identified as having immunosuppressive activity. Representative compounds include triptolide, 16-hydroxytriptolide, triptophenolide, tripdiolide, and tripchlorolide, as described, for example, in Lipsky *et al*. (1994) and Zheng *et al.* (1991; 1994).

The administration and therapeutic effectiveness of these compounds have been limited, however, by their low water solubility. This problem has been addressed by formulating the compounds in mixtures of ethanol and polyethoxylated castor oil (*e.g. ,* "CREMOPHOR EL^{™}"), allowing subsequent dilution in saline for intravenous administration. However, such formulations have suffered from high toxicity, due to the high concentration of solubilizing agent required to dissolve these compounds. For example, the ratio of solubilizing agent (ethanol plus "CREMOPHOR EL^{™}") to triptolide in such formulations is typically on the order of 1000:1 or greater, due to the poor solubility of triptolide (Morris, 1991; Morris *et al*., 1991). Standardization of dosage amounts is also more problematic with a suspension than with a solution.

It is therefore desirable to provide immunosuppressive compounds having comparatively low toxicity and improved water solubility. Ideally, such compounds would show immunosuppressive activity in their water soluble form, or would be convertible to an immunosuppressive form *in vivo.*

In one aspect, the invention provides compounds which are useful as prodrugs for immunosuppressive and anti-inflammatory therapy. The compounds are derivatives of triptolide having hydrophilic substituents, represented by structure III, as shown and described below. The compounds possess greater water solubility than the non-derivatized parent compound, triptolide, and are effective to hydrolytically convert to the parent compound *in vivo.*

These and other objects and features of the invention will become more fully apparent when the following detailed description of the invention is read in conjunction with the accompanying drawings.

Fig. 1 shows the preparation of an epoxy ring-opened derivative of triptolide, in accordance with structure III; and

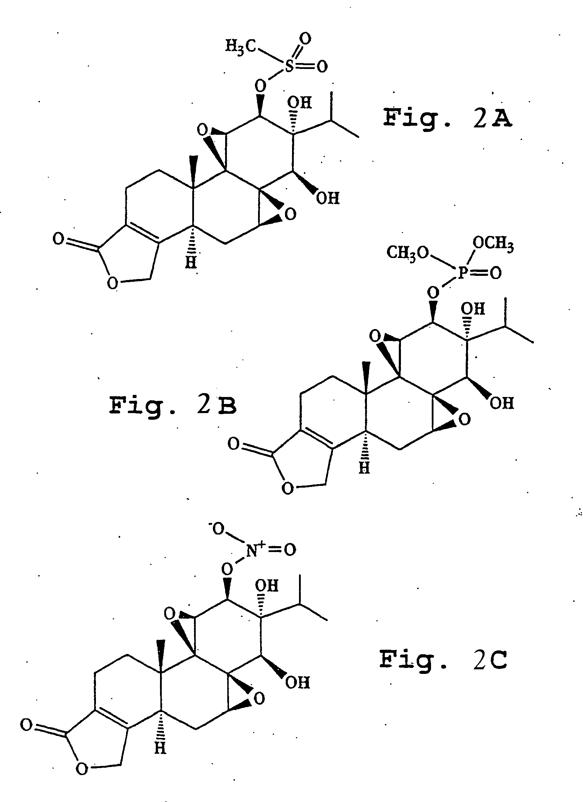

Figs. 2A-2C show examples of specific embodiments of structure III.

### I. Definitions

The terms below have the following meanings unless indicated otherwise.

"Alkyl" refers to a fully saturated acyclic monovalent radical containing carbon and hydrogen, and which may be branched or a straight chain. Examples of alkyl groups are methyl, ethyl, n-butyl, t-butyl, n-heptyl, and isopropyl. "Cycloalkyl" refers to a fully saturated cyclic monovalent radical containing carbon and hydrogen, which may be further substituted with alkyl. Examples of cycloalkyl groups are cyclopropyl, methyl cyclopropyl, cyclobutyl, cyclopentyl, ethylcyclopentyl, and cyclohexyl, "Lower alkyl" refers to an alkyl radical of one to six carbon atoms, as exemplified by methyl, ethyl, n-butyl, i-butyl, t-butyl, isoamyl, n-pentyl, and isopentyl.

"Alkenyl" refers to a monovalent or divalent unsaturated, preferably mono-unsaturated, radical containing carbon and hydrogen, and which may be cyclic, branched or a straight chain. "Lower alkenyl" refers to such a radical having one to four carbon atoms.

A "triptolide derivative" or "triptolide analog," as described herein, refers to a compound based on triptolide, 16-hydroxytriptolide or tripdiolide (2-hydroxytriptolide) which is derivatized at the 12,13-epoxy group or at the lactone ring of the parent compound.

For the purposes of the current disclosure, the following numbering scheme is used for triptolide and triptolide analogs:

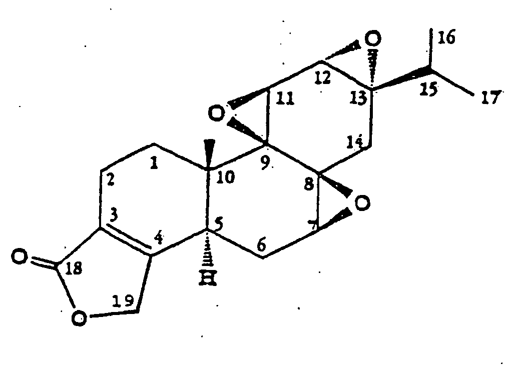

### II. Triptolide Analogs

This section describes the preparation of compounds as represented by structure III and described further below. The present compounds, which are derivatives of triptolide having hydrophilic substituents, possess greater water solubility than the non-derivatized starting compound, and are effective to hydrolyze and convert *in vivo* to the parent compound. The compounds are useful as prodrugs for immunosuppressive and anti-inflammatory applications.

The present invention relates to a
compound having the structure III: 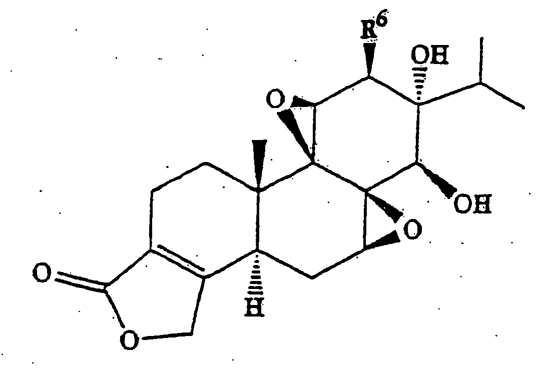 wherein
R⁶ is a leaving group selected from the group consisting of alkyl sulfonate, fluoroalkyl sulfonate, aryl sulfonate, fluorosulfonate, nitrate, alkyl phosphate, alkyl borate, trialkylammonium, and dialkylsulfonium.

The compounds of the invention may be prepared from triptolide, as obtained from the root xylern of the Chinese medicinal plant *Tripterygium wilfordii* (TW) or from other known sources. The TW plant is found in the Fujiang Province and other southern provinces of China; TW plant material can generally be obtained in China or through commercial sources in the United States. Methods for preparing triptolide and some of its derivatives (e.g. tripdiolide and 16-hydroxytriptolide) are known in the an and are described, for example, in Kupchan *et al*. (1972, 1977); Lipsky *et al*. (1994); Pu *et al*. (1990); and Ma *et al*. (1992).

The triptolide analogs have the structure III, as shown above, where R⁶ is a leaving group selected from the group consisting of alkyl sulfonate, fluoroalkyl sulfonate, aryl sulfonate, fluorosulfonate, nitrate, alkyl phosphate, alkyl borate, trialkylammonium, and dialkylsulfonium. Preferred leaving groups are tosylate, mesylate, fluorosulfonate, trifluoromethylsulfonate, nitrate, and alkyl phosphates or boronates, represented by -OP(O)(OR⁴)₂, and -OB(OR⁴)₂, where R⁴ is hydrogen or lower alkyl. The group OR³ is -OH or -O-(C=O)-R, where R is lower alkyl.

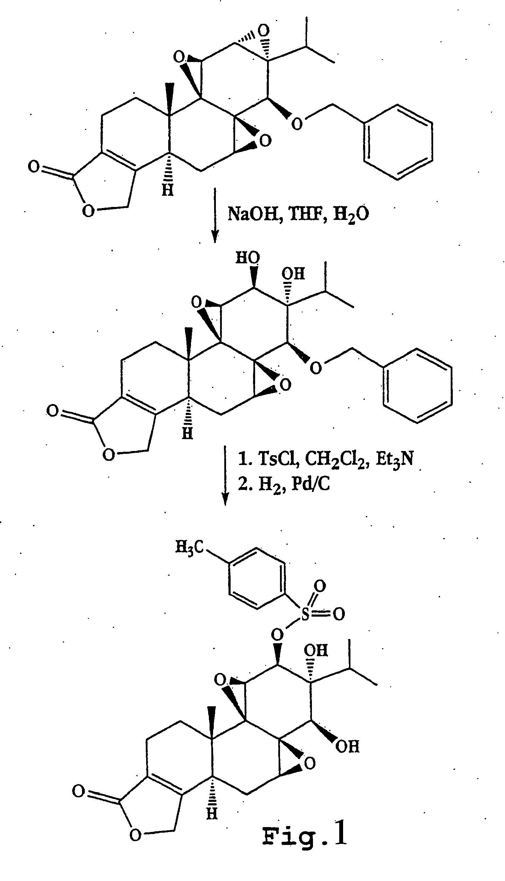

These compounds are prepared by base-catalyzed ring opening of the 12,13 epoxy group of triptolide, as illustrated in Fig. 1. As noted in Yu *et al*., the 12,13 epoxide of triptolide is less sterically hindered and reacts more readily than the 7,8 and 9,11 epoxides. The epoxide is regenerated *in vivo* by displacement of the 12-leaving group, restoring the triptolide structure.

In the synthesis shown in Fig. 1, the hydroxyl at C14 is first protected as a benzyl ether, which is later removed by hydrogenation. The nucleophile, typically hydroxide ion, attacks at the less hindered 12-carbon of the epoxide. The resulting 12-hydroxyl group of the 1,2-diol is then converted to the leaving group R⁶, in this case a tosylate. Other examples of compounds of structure III are shown in Figs, 2A-2C.

If desired, the 13-hydroxyl (and 14-hydroxyl) may be converted to a hydrolyzable group, so that the compound will be more stable upon storage, but will still convert (albeit more slowly) to the epoxide *in vivo.* Because the 13-hydroxyl is tertiary and thus slow to react, use of an unhindered, reactive acylating agent, e.g., acetyl chloride, is preferred.

### III. Therapeutic Compositions

Formulations containing the triptolide analogs of the invention may take the form of solid, semi-solid, lyophilized powder, or liquid dosage forms, such as tablets, capsules, powders, sustained-release formulations, solutions, suspensions, emulsions, ointments, lotions, or aerosols, preferably in unit dosage forms suitable for simple administration of precise dosages. The compositions typically include a conventional pharmaceutical carrier or excipient and may additionally include other medicinal agents, carriers, or adjuvants. Preferably, the composition will be about 0.5 % to 75 % by weight of a compound or compounds of the invention, with the remainder consisting of suitable pharmaceutical excipients. For oral administration, such excipients include pharmaceutical grades of mannitol, lactose, starch, magnesium stearate, sodium saccharine, talcum, cellulose, glucose, gelatin, sucrose, magnesium carbonate, and the like. If desired, the composition may also contain minor amounts of non-toxic auxiliary substances such as wetting agents, emulsifying agents, or buffers.

The composition may be administered to a subject orally, transdermally or parenterally, *e*.*g*., by intravenous, subcutaneous, intraperitoneal, or intramuscular injection. For use in oral liquid preparation, the composition may be prepared as a solution, suspension, emulsion, or syrup, being supplied either in liquid form or a dried form suitable for hydration in water or normal saline. For parenteral administration, an injectable composition for parenteral administration will typically contain the iriptolide analog in a suitable intravenous solution, such as sterile physiological salt solution.

Liquid compositions can be prepared by dissolving or dispersing the triptolide analog (about 0.5% to about 20%) and optional pharmaceutical adjuvants in a carrier, such as, for example, aqueous saline, aqueous dextrose, glycerol, or ethanol, to form a solution or suspension. The high water solubility of the compounds of the invention make them particularly advantageous for administering in aqueous solution, *e.g.* by intraperitoneal injection. Although aqueous solutions are preferred, compositions in accordance with the invention may also be formulated as a suspension in a lipid (*e.g.*, a triglyceride, a phospholipid, or a polyethoxylated castor oil such as "CREMOPHOR EL^{™}"), in a liposomal suspension, or in an aqueous emulsion.

The compound may also be administered by inhalation, in the form of aerosol particles, either solid or liquid, preferably of respirable size. Such particles are sufficiently small to pass through the mouth and larynx upon inhalation and into the bronchi and alveoli of the lungs. In general, particles ranging from about 1 to 10 µm (microns) in size, and preferably less than about 5 µm (microns) in size, are respirable. Liquid compositions for inhalation comprise the active agent dispersed in an aqueous carrier, such as sterile pyrogen free saline solution or sterile pyrogen free water. If desired, the composition may be mixed with a propellant to assist in spraying the composition and forming an aerosol.

Methods for preparing such dosage forms are known or will be apparent to those skilled in the art; for example; see Remington's Pharmaceutical Sciences (19th Ed., Williams & Wilkins, 1995). The composition to be administered will contain a quantity of the selected compound in a pharmaceutically effective amount for effecting immunosuppression in a subject.

### IV. Therapeutic Uses

The compositions of the present invention are useful in applications for which triptolide has proven effective, particularly in immunosuppression therapy, as in treating an autoimmune disease, preventing transplantation rejection, or treating or preventing graft-versus-host disease (GVHD). Triptolide and the present analogs are also useful for treatment of other inflammatory conditions, such as traumatic inflammation, and in reducing male fertility.

Common autoimmune diseases which are appropriate for immunotherapy include asthma, atherosclerosis, Type I diabetes, multiple sclerosis, psoriasis, systemic lupus erythematosis (SLE), rheumatoid arthritis, and various allergies. In treating an autoimmune condition, the patient is given the composition on a periodic basis, *e*.*g*., 1-2 times per week, at a dosage level sufficient to reduce symptoms and improve patient comfort.

The dose that is administered is preferably in the range of 1-25 mg/kg patient body weight per day, with lower amounts being preferred for parenteral administration, and higher amounts being preferred for oral administration. Optimum dosages can be determined by routine experimentation according to methods known in the art.

Immunosuppressive activity of compounds *in vivo* can be evaluated by the use of established animal models known in the art. Such assays may be used to evaluate the relative effectiveness of immunosuppressive compounds and to estimate appropriate dosages for immunosuppressive treatment. These assays include, for example, a well-characterized rat model system for allografts, described by Ono and Lindsey (1969), in which a transplanted heart is attached to the abdominal great vessels of an allogeneic recipient animal, and the viability of the transplanted heart is gauged by the heart's ability to beat in the recipient animal. A xenograft model, in which the recipient animals are of a different species, is described by Wang (1991) and Murase (1993). A model for evaluating effectiveness against GVHD involves injection of normal F₁ mice with parental spleen cells; the mice develop a GVHD syndrome characterized by splenomegaly and immunosuppression (Korngold, 1978; Gleichmann, 1984). Single cell suspensions are prepared from individual spleens, and microwell cultures are established in the presence and absence of concanavalin A to assess the extent of mitogenic responsiveness.

For therapy in transplantation rejection, the method is intended particularly for the treatment of rejection of heart, kidney, liver, cellular, and bone marrow transplants, and may also be used in the treatment of GVHD. The treatment is typically initiated perioperatively, either soon before or soon after the surgical transplantation procedure, and is continued on a daily dosing regimen, for a period of at least several weeks, for treatment of acute transplantation rejection. During the treatment period, the patient may be tested periodically for immunosuppression level, *e*.*g*., by a mixed lymphocyte reaction involving allogenic lymphocytes, or by taking a biopsy of the transplanted tissue.

In addition, the composition may be administered chronically to prevent graft rejection, or in treating acute episodes of late graft rejection. As above, the dose administered is preferably 1-25 mg/kg patient body weight per day, with lower amounts being preferred for parenteral administration, and higher amounts for oral administration. The dose may be increased or decreased appropriately, depending on the response of the patient, and over the period of treatment, the ability of the patient to resist infection.

For treating rheumatoid arthritis, the composition may be administered by intravenous injection or by direct injection into the affected joint. The patient may be treated at repeated intervals of at least 24 hours, over a several week period following the onset of symptoms of the disease in the patient.

The compounds are also useful as potentiators when administered concurrently with another immunosuppressive drug for immunosuppressive treatments as discussed above. A conventional immunosuppressant drug, such as cyclosporin A, FK506, azathioprine, rapamycin, mycophenolic acid, or a glucocorticoid, may thus be administered in an amount substantially less (e.g. 20% to 50% of the standard dose) than when the compound is administered alone. Alternatively, the triptolide analog and immunosuppresive drug are administered in amounts such that the resultant immunosuppression is greater than what would be expected or obtained from the sum of the effects obtained with the drug and triptolide analog used alone. Typically, the immunosuppressive drug and potentiator are administered at regular intervals over a time period of at least 2 weeks.

### EXAMPLES

The following example is intended to illustrate but not in any way limit the invention. While the invention has been described with reference to specific methods and embodiments, it will be appreciated that various modifications may be made without departing from the invention.

### Example 1

### Synthesis of 12-tosyloxy-13-hydroxy triptolide (Fig. 1)

The 14-hydroxyl group is first protected by conversion to a benzyl ether. To avoid reaction of the acidic hydrogens of the conjugated lactone with basic reagents, such as metal hydrides, the compound (1 eq) is reacted with BzBr (2.5 eq) in the presence of Ag₂O (2 eq) in DMF under an inert atmosphere at 0°C (see, for example, Mori et al.). The mixture is allowed to come to room temperature with stirring and stirred for about 24 h. The mixture is diluted with ether, washed with water and brine, dried over anhydrous MgSO₄ and concentrated. The residue is purified, if desired, by chromatography on silica gel.

The resulting 14-O-benzyl triptolide is then heated with NaOH in aqueous THF to convert the 12,13 epoxide to the diol. The solution is concentrated and the residue taken up in ether and worked up as above.

The diol (1 eq) is dissolved in CH₂Cl₂, and a solution of TsCl (1.5 eq) and triethylamine (1.5 eq) in the same solvent is added. After the reaction is complete by TLC, the mixture is washed with water and brine, dried over anhydrous MgSO₄, and concentrated. The residue is purified by silica gel chromatography.

The resulting benzyl ether-protected tosylate is dissolved in dry THF, 5 % Pd/C (approx. 25 mg/meq of substrate) is added, and the mixture is purged with H₂ (atmospheric pressure) and stirred at room temperature until deprotection is complete, approx. 2-3 hrs. The solution is then filtered and concentrated to obtain the product.

## Claims

1. A compound having the structure III: 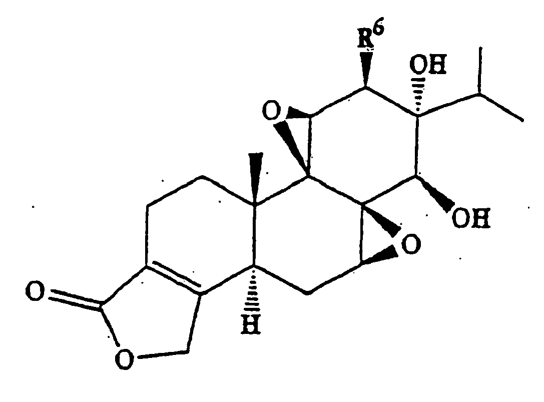 wherein
R⁶ is a leaving group selected from the group consisting of alkyl sulfonate, fluoroalkyl sulfonate, aryl sulfonate, fluorosulfonate, nitrate, alkyl phosphate, alkyl borate, trialkylammonium, and dialkylsulfonium.

2. A compound in accordance with claim 1, wherein R⁶ is selected from nitrate, tosylate, mesylate, fluorosulfonate, trifluoromethylsulfonate, -OP(O)(OR⁴)₂, and -GB(OR⁴)₂, where R⁴ is hydrogen or C₁ to C₆ alkyl.

3. A pharmaceutical composition comprising at least one compound as defined in claim 1 or 2.

4. The pharmaceutical composition of claim 3 comprising 0.5 wt.% to 75 wt.% of one or more compounds of claim 1 or 2.

5. Use of a compound as defined in claim 1 or 2 for the preparation of a pharmaceutical composition for use in an immunosuppression therapy, for treating an autoimmune disease, preventing transplantation rejection, treating or preventing graft-versus-host disease, treatment of inflammatory conditions or for reducing male fertility.

## Patentansprüche

1. Verbindung der Struktur III: 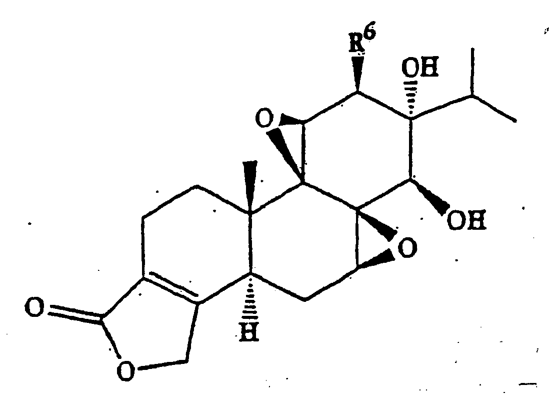 wobei
R⁶ eine Abgangsgruppe ist, die aus Alkylsulfonat, Fluoralkylsulfonat, Arylsulfonat, Fluorsulfonat, Nitrat, Alkylphosphat, Alkylborat, Trialkylammonium und Dialkylsulfonium ausgewählt ist.

2. Verbindung nach Anspruch 1, wobei R⁶ aus Nitrat, Tosylat, Mesylat, Fluorsulfonat, Trifluormethylsulfonat, -OP(O)(OR⁴)₂ und -OB(OR⁴)₂ ausgewählt ist, wobei R⁴ Wasserstoff oder C₁- bis C₆-Alkyl ist.

3. Arzneimittel, umfassend mindestens eine Verbindung wie in Anspruch 1 oder 2 definiert.

4. Arzneimittel nach Anspruch 3, umfassend 0,5 Gew.-% bis 75 Gew.-% einer oder mehrerer Verbindungen gemäß Anspruch 1 oder 2.

5. Verwendung einer Verbindung, wie in Anspruch 1 oder 2 definiert, zur Herstellung eines Arzneimittels zur Verwendung in einer Immunsuppressionstherapie, zur Behandlung einer Autoimmunerkrankung, Vorbeugung von Transplantatabstoßung, Behandlung oder Vorbeugung von Transplantat-Wirt-Reaktion, Behandlung von entzündlichen Zuständen oder zur Reduzierung von männlicher Fruchtbarkeit.

## Revendications

1. Composé ayant la structure III : 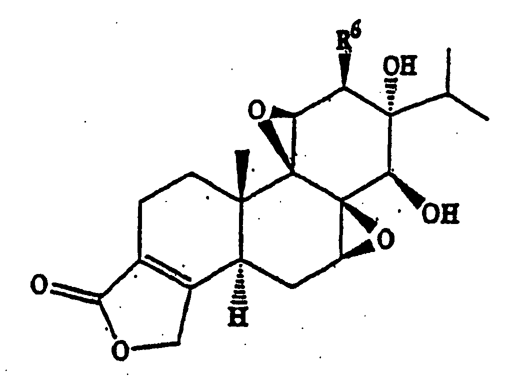 dans laquelle R⁶ est un groupe partant choisi parmi le groupe constitué du sulfonate d'alkyle, du sulfonate de fluoroalkyle, du sulfonate d'aryle, du fluorosulfonate, du nitrate, du phosphate d'alkyle, du borate d'alkyle, du trialkylammonium et du dialkylsulfonium.

2. Composé selon la revendication 1, dans lequel R⁶ est choisi parmi le nitrate, le tosylate, le mesylate, le fluorosulfonate, le trifluorométhylsulfonate, -OP(O)(OR⁴)₂ et -OB(OR⁴)₂ où R⁴ est un atome d'hydrogène ou un alkyle en C₁ à C₆.

3. Composition pharmaceutique comprenant au moins un composé tel que défini dans la revendication 1 ou 2.

4. Composition pharmaceutique de la revendication 3, comprenant 0,5 % pds à 75 % pds d'un ou plusieurs composés de la revendication 1 ou 2.

5. Utilisation d'un composé tel que défini dans la revendication 1 ou 2 pour la préparation d'une composition pharmaceutique destinée à être utilisée dans une thérapie d'immunosuppression, pour le traitement d'une maladie autoimmune, la prévention d'un rejet de transplantation, le traitement ou la prévention d'une maladie du greffon contre l'hôte, le traitement d'états inflammatoires ou pour la réduction de la fertilité masculine.
